# EUROPEAN PATENT APPLICATION

(11) **EP 2 489 357 A1**
(43) Date of publication of application: **22.08.2012**
(21) Application number: 12168434.4
(22) Date of filing: 20.09.2007
(51) Int. Cl.: A61K 33/38, A61K 33/00, A01N 55/02, A61K 31/28, A61K 31/085, A61K 45/06

(54) **Novel Triclosan Salts**

(30) Priority: 20.09.2006 US 846272 P
(62) Divisional of application: 07838598.6
(71) Applicant: Tyco Healthcare Group, LP, North Haven, CT 06473 (US)
(72) Inventor: Stopek, Joshua, Yalesville, CT Connecticut 06492 (US); Cuevas, Brian, Cumming, GA Georgia 30040 (US); Belcheva, Nadya, Hamden, CT Connecticut 06518 (US)
(74) Representative: Soames, Candida Jane

(57) **Abstract**

An article comprising a silver-triclosan salt, wherein the article is selected from the group consisting of medical devices and packaging materials, and wherein the triclosan is present in an amount from about 40% to about 99% of the weight of the salt and the silver is present in an amount from about 1 to about 60% by weight of the salt.

## Description

### BACKGROUND

The present disclosure relates generally to novel triclosan salts. The triclosan salts may include a silver ion. In embodiments the triclosan salts may be utilized as an antimicrobial composition in the synthesis, coating or modification of medical devices and packaging materials, as well as textile products. In other embodiments the triclosan salts may be utilized by themselves or in combination with a pharmaceutically acceptable carrier as pharmaceutical compositions.

The use of antimicrobial agents on textiles is known. See, e.g., U.S. Patent Application Publication No. 2003/0204916. The use of antimicrobial agents on medical devices such as sutures and/or packages containing said sutures has also been previously disclosed. However, some medical devices may not provide effective levels of antimicrobial activity for a sufficient period of time. Moreover, as is apparent from U.S. Patent Publication Nos. 2004/0068293 and 2004/0068294, antimicrobial agents on medical devices can be undesirably transferred to their packages, requiring the use of higher levels of antimicrobial agents in order to obtain the desired antimicrobial effect upon implantation of the suture or other medical device in vivo.

Accordingly, there is a need for medical devices, packaging materials and textiles that can retain enhanced antimicrobial efficacy. There is also a need for an easy and inexpensive method of applying antimicrobial agents to a medical device, packaging material or textile that provides protection against microorganisms for extended periods of time, with minimal loss of the antimicrobial agents from the article surface and/or minimal transference of the antimicrobial agent to packaging materials, etc. In this way, lower amounts of antimicrobial agents may be utilized to achieve the desired antimicrobial effect.

### SUMMARY

The present disclosure provides novel salts having triclosan and silver, wherein the triclosan is present in an amount from about 40 to about 99% by weight of the salt and the silver is present in an amount from about 1 to about 60% by weight of the salt. Pharmaceutical compositions including these silver-triclosan salts, optionally in a pharmaceutically acceptable carrier, are also provided.

The present disclosure also provides articles including these silver-triclosan salts. Suitable articles include medical devices, packaging materials and textiles. The articles may have the silver-triclosan salts incorporated therein or the silver-triclosan salt may be present in a coating on the article.

Methods for making these silver-triclosan salts are also provided. Such methods include combining triclosan with at least one base to form a first triclosan salt and combining a silver compound with the first triclosan salt to form the silver-triclosan salt.

### DELAMED DESCRIPTION OF THE EMBODIMENTS

In accordance with the present disclosure, novel salts are provided which are based upon triclosan and silver. The salts of the present disclosure may be used in the synthesis, coating, or modification of various articles. As used herein, "articles" include, but are not limited to, medical devices, packaging materials and textiles. The salts of the present disclosure may also be used by themselves or in combination with a pharmaceutically acceptable carrier as pharmaceutical compositions.

As used herein, "triclosan," also known as 2,4,4'-trichloro-2'-hydroxydiphenyl ether, includes any derivative thereof. Derivatives of triclosan which may be used in the present disclosure further include those compounds in which one or both of the phenyl groups may be substituted by one or more substituent groups in addition to the chloro substituents already present on the phenyl rings. Examples of suitable substituents are alkyl groups containing from about 1 to about 4 carbon atoms, haloalkyl groups containing from about 1 to about 4 carbon atoms, alkoxy groups containing from about 1 to about 4 carbon atoms, cyano, allyl, amino and acetyl groups, and combinations thereof. In some embodiments, the triclosan may be substituted with methyl, methoxy or trifluoromethyl groups. It will be understood that if triclosan is substituted by more than one substituent, then the substituents may be the same or different.

In some embodiments an ester of triclosan or an ester of a triclosan derivative may be used in the present disclosure. Examples of suitable esters include phosphate, phosphonate, sulfate, glucuronide, succinate and glutamate esters. In some embodiments, a phosphate ester of triclosan or a triclosan derivative may be utilized. Phosphate esters may be prepared by the phosphorylation of triclosan, using methods within the purview of those skilled in the art.

In accordance with the present disclosure, a first triclosan salt may be formed by reacting triclosan with a base. Suitable bases which may be utilized include, for example, sodium hydroxide, potassium hydroxide, calcium hydroxide, and/or magnesium hydroxide, and basic salts in aqueous solution such as sodium bicarbonate, sodium carbonate, calcium carbonate, potassium carbonate and potassium bicarbonate.

In embodiments, triclosan may be reacted with a base such as sodium hydroxide to form a sodium-triclosan salt. Amounts utilized in forming such a sodium-triclosan salt are within the purview of one skilled in the art. In some embodiments, from about 5 to about 95 percent by weight of triclosan and from about 95 to about 5 percent by weight of sodium hydroxide may be combined to form the sodium-triclosan salt, in other embodiments from about 30 to about 70 percent by weight of triclosan and from about 70 to about 30 percent by weight of sodium hydroxide may be combined to form the sodium-triclosan salt.

Once the first triclosan salt has been prepared, e.g., a sodium-triclosan salt, it may then be reacted with a silver compound such as a silver salt to form a silver-triclosan salt. Suitable silver compounds which may be reacted with the sodium-triclosan salt include, for example, silver salts such as silver acetate, silver ascorbate, silver benzoate, silver bromide, silver carbonate, silver chloride, silver citrate, silver gluconate, silver iodate, silver iodide, silver lactate, silver laurate, silver nitrate, silver oxide, silver palmitate, silver phosphate, silver propionate, silver salicylate, silver sulfate, silver laurel sulfate, and mixtures and combinations thereof. Other silver compounds which may be reacted with the sodium-triclosan salt to form the silver-triclosan salt include, for example, colloidal silver, silver protein, silver sulfadiazine, silver arsphenamine, zinc-silver allantoinate, and mixtures and combinations thereof, optionally together with any of the above-mentioned silver salts.

Methods for adding the silver compound to the first triclosan salt are within the purview of those skilled in the art. The amount of silver compound added to the first triclosan salt may vary, but can be from about 5 to about 95 percent by weight, in embodiments from about 20 to about 80 percent by weight, typically from about 40 to about 60 percent by weight, while the amount of first triclosan salt can be from about 95 to about 5 percent by weight, in embodiments from about 80 to about 20 percent by weight, typically from about 60 to about 40 percent by weight.

In embodiments, the silver compound may be added to the first triclosan salt in solution. For example, as noted above, triclosan may be added to a base such as sodium hydroxide, forming a sodium-triclosan salt in solution. To this solution the silver compound may be added, thus forming the silver-triclosan salt in solution. In some embodiments, the combined silver compound, base and triclosan may be stirred or mixed to enhance formation of the silver-triclosan salt. In other embodiments, the combination may be heated to a temperature from about 60°C to about 180°C, in embodiments from about 70°C to about 150°C, typically from about 80°C to about 130°C. After a suitable period of time, in embodiments from about 2 minutes to about 24 hours, in embodiments from about 2 hours to about 18 hours, in other embodiments from about 4 hours to about 12 hours, the silver-triclosan salt may be recovered by methods within the purview of those skilled in the art, for example, by distillation, filtration, centrifugation, and the like.

Once obtained, triclosan may be present in the resulting silver-triclosan salt in an amount from about 40 to about 99 % by weight of the total weight of the silver-triclosan salt, in embodiments from about 50 to about 90% by weight of the total weight of the silver-triclosan salt, typically from about 60 to about 80% by weight of the total weight of the silver-triclosan salt. The silver ion may be present in an amount from about 1 to about 60% by weight of the total weight of the silver-triclosan salt, in embodiments from about 10 to about 50% by weight of the total weight of the silver-triclosan salt, typically from about 20 to about 40% by weight of the total weight of the silver-triclosan salt.

In some embodiments, the silver-triclosan salt may be further complexed with other soluble salts or ions. For example, chlorhexidine and its salts may be combined with the silver-triclosan salt to form a silver-chlorhexidine-triclosan complex. Suitable chlorhexidine salts which may be utilized include, but are not limited to, chlorhexidine acetate, chlorhexidine gluconate, chlorhexidine hydrochloride, chlorhexidine sulfate, and mixtures or combinations thereof.

Other salts which may be further complexed with the silver-triclosan salt may possess cationic ions including, but not limited to, calcium, sodium, lithium, aluminum, magnesium, potassium, manganese, copper, zinc, platinum, gold, cerium, gallium, osmium, and the like. Such salts may possess anions including, but not limited to, acetates, ascorbates, benzoates, bitartrates, bromides, brominated furanones, carboxylates, carbonates, chlorides, citrates, folates, gluconates, iodates, iodides, lactates, laurates, oxalates, oxides, palmitates, perborates, phenosulfonates, phosphates, propionates, salicylates, stearates, succinates, sulfadiazines, sulfates, sulfides, sulfonates, tartrates, thiocyanates, thioglycolates, thiosulfates, and the like.

As noted above, articles which may be treated with the compositions of the present disclosure include medical devices, packaging materials and textiles. Any medical device may be treated with the salts of the present disclosure. Suitable medical devices include, for example, staples, clips, drug delivery devices, stents, pins, screws, and fibrous surgical articles such as sutures, prosthetic ligaments, prosthetic tendons, woven mesh, gauze, dressings, growth matrices, and the like.

Packaging materials include, but are not limited to, packaging materials for products such as medical devices, pharmaceuticals, textiles, consumer goods, foods, and the like, or any other article in which it may be desirable to minimize or inhibit bacterial colonization.

Suitable textiles include textiles utilized for personal hygiene and health care products, commodity plastics, fibers, woven materials, clothing, etc. Textiles may include natural fibers such as cotton, wool, and the like, or synthetic fibers such as polyesters, polyamides, polyolefins, and the like. Textiles may also include blends or mixtures of natural fibers, blends or mixtures of synthetic fibers, or blends or mixtures of natural fibers with synthetic fibers. Suitable synthetic fibers include, but are not limited to, polyolefins such as polyethylene, polypropylene, and polybutylene; halogenated polymers such as polyvinyl chloride; polyesters such as polyethylene terephthalate; polyesters/polyethers; polyamides such as nylon 6 and nylon 6,6; polyurethanes; as well as homopolymers, copolymers, or terpolymers of any of the foregoing, as well as any combinations thereof, and the like.

In embodiments, the textile may be dyed or colored with any type of colorant, such as, for example, poly(oxyalkylenated) colorants, as well as pigments, dyes, tints, and the like. Other additives may also be present on and/or within the textile, including antistatic agents, brightening compounds, nucleating agents, antioxidants, UV stabilizers, fillers, permanent press finishes, softeners, lubricants, curing accelerators, and the like.

Textiles may be of any standard construction, including knit, woven, or non-woven forms. The textiles may be utilized in any suitable application including, without limitation, apparel, upholstery, bedding, wiping cloths, towels, gloves, rugs, floor mats, drapery, napery, bar runners, textile bags, awnings, vehicle covers, boat covers, tents, and the like.

Once formed, the silver-triclosan salt of the present disclosure may be incorporated into the material utilized to form the article to be treated, such as a medical device, packaging material, textile, etc., or applied as a coating to such material. For example, where the article to be treated is polymeric, the silver-triclosan salt may be mixed or blended with the polymer prior to forming the article to be treated, thus becoming incorporated within the article itself. Methods for adding materials like the silver-triclosan salt of the present disclosure to polymers are within the purview of one skilled in the art and include conventional mixing equipment such as Brabender or Banbury mixers, extruders, mills, and the like.

Where the silver-triclosan salt of the present disclosure is incorporated into the polymeric material utilized to form an article, i.e., a medical device, packaging material, or textile, the amount of the silver-triclosan salt applied should be present in an effective amount to provide antimicrobial properties to the article. The exact amount will depend upon the configuration of the article, the specific silver-triclosan salt utilized, and the method of incorporation in the polymeric article. In embodiments, the silver-triclosan salt of the present disclosure may be present in amounts from about 0.001 to about 25 percent by weight of the article, in embodiments from about 0.01 to about 15 percent by weight of the article, typically from about 0.1 to about 5 percent by weight of the article.

As noted above, in other embodiments the silver-triclosan salt of the present disclosure may be applied as a coating to an article to be treated. In some embodiments, triclosan may be added to a base solution, such as sodium hydroxide, and silver may be added to the combination of the two whereby the silver-triclosan salt is formed in an antimicrobial solution. In other embodiments, the silver-triclosan salts of the present disclosure may be first formed and then added to a separate solvent to form an antimicrobial solution, which may then be utilized to coat an article to be treated.

The antimicrobial solution can include any solvent or combination of solvents suitable for the silver-triclosan salt. To be suitable, the solvent must (1) be miscible with the silver-triclosan salt and (2) not appreciably affect the integrity of any material used to form the medical device. As noted above, in embodiments the solvent utilized may include the base utilized to form the silver-triclosan salt. In other embodiments, the solvent utilized may be a polar solvent. Some examples of suitable solvents include methylene chloride, chloroform, ethyl acetate, methyl acetate, N-methyl 2-pyrrolidone, 2-pyrrolidone, propylene glycol, tetrahydrofuran (THF), acetone, oleic acid, methyl ethyl ketone, water, and mixtures thereof. In one embodiment, methylene chloride may be used as a solvent.

The antimicrobial solution generally contains from about 0.001 to about 25% of the silver-triclosan salt by weight. The exact amount of the silver-triclosan salt will depend on a number of factors, such as the article being contacted, the choice of solvent employed, and any other additives utilized. In one embodiment, the silver-triclosan salt may be present in an amount from about 0.01% to about 15% by weight of the antimicrobial solution, in embodiments from about 0.1 % to about 5% by weight of the antimicrobial solution.

The method of preparing the antimicrobial solution of the present disclosure is not critical and can be a relatively simple procedure. For example, the silver-triclosan salt in solution and any other additive may be combined with mixing at room temperature to produce the antimicrobial solution. In some useful embodiments, the solvent may be heated to enhance formation of the antimicrobial solution, provided that significant degradation of the antimicrobial activity of the silver-triclosan salt is avoided.

Without wishing to be bound by any theory, it is believed that the combined triclosan and silver of the silver-triclosan salt enhance the solubility of both the triclosan and the silver ions. By enhancing the solubility of the triclosan and silver ions in the antimicrobial solution, lower amounts of triclosan and silver are required to obtain the desired amount of triclosan and silver upon the article being treated, which reduces the amount of triclosan and silver required to achieve the desired antimicrobial effect.

Any known technique may be employed for applying the antimicrobial solution to the article to be treated, whether it be a medical device, packaging material, or textile. Suitable techniques include dipping, spraying, wiping and brushing. Since the antimicrobial solution contains a solvent, a curing step may be employed in embodiments to remove the solvent, leaving the silver-triclosan salt on the article. Suitable curing steps for removal of the solvent include, but are not limited to, evaporation and/or lyophilization. Upon removal of the solvent, the silver-triclosan salt remains bound to the article being treated.

Where applied as a coating to an article, i.e., a medical device, packaging material, or textile, the amount of the silver-triclosan salt applied should be present in an effective amount to provide antimicrobial properties to the article. The exact amount will depend upon the configuration of the article, the specific silver-triclosan salt utilized, and the method of application.

For medical devices, the amount of silver-triclosan salt as a coating may be from about 0.001 to about 25 percent by weight of the medical device, in embodiments from about 0.01 to about 15 percent by weight of the medical device, typically from about 0.1 to about 5 percent by weight of the medical device. For a suture, the silver-triclosan salt will be applied so that it is present in an amount from about 0.001 to about 25 percent by weight of the suture, in embodiments from about 0.01 to about 15 percent by weight of the suture, typically from about 0.1 to about 5 percent by weight of the suture.

Moreover, the silver-triclosan salt of the present disclosure may be applied as a coating to packaging materials for medical devices or other articles in amounts from about 0.001 to about 25 percent by weight of the packaging material, in embodiments from about 0.01 to about 15 percent by weight of the packaging material, typically from about 0.1 to about 5 percent by weight of the packaging material.

In other embodiments, as noted above, the silver-triclosan salt may be applied as a coating to textiles, including those utilized for personal hygiene and health care products, commodity plastics, fibers, woven materials, clothing, etc. The silver-triclosan salt of the present disclosure may be applied as a coating to such textiles in amounts from about 0.001 to about 25 percent by weight of the textile, in embodiments from about 0.01 to about 15 percent by weight of the textile, typically from about 0.1 to about 5 percent by weight of the textile.

In some embodiments, the antimicrobial solution may also contain at least one adherence-enhancing agent which enhances the affinity of the silver-triclosan salt for the article being coated with the silver-triclosan salt. Suitable adherence-enhancing agents include, but are not limited to, N-methylglucamine; L-arginine; sodium lauryl sulfate; cyclodextrins such as beta-cyclodextrin and hydroxypropyl-beta-cyclodextrin; ethanolamines such as ethanolamine, triethanolamine, and diethanolamine; and benzoates such as sodium benzoate and sodium methyl 4-hydroxybenzoate.

Where utilized, the adherence-enhancing agent may be present in amounts from about 1 percent to about 10 percent by weight of the antimicrobial solution, typically from about 2 percent to about 8 percent by weight of the antimicrobial solution, more typically from about 4 percent to about 6 percent by weight of the antimicrobial solution.

The choice of adherence-enhancing agent utilized in the present disclosure may depend upon the selected silver-triclosan salt and the article to which it may be applied. In some embodiments a micellular complex could be formed between the silver-triclosan salt and the adherence-enhancing agent. For example, where the antimicrobial solution includes a silver-triclosan salt combined with a cyclic sugar derivative such as a cyclodextrin, a micellular complex may form between the silver-triclosan salt and cyclodextrin which will remain on the surface of the article being coated upon removal of the solvent. The silver-triclosan salt, in this case the micellular complex, will not migrate through the article being coated and the hydrophilic portion of the silver-triclosan salt, i.e., the micelle, will have greater affinity for the article being coated than the silver-triclosan salt alone, especially where the article being coated is made of a polyester or possesses a synthetic film-forming coating as described above.

Articles of the present disclosure may also possess an additional coating to enhance their physical properties. Many suitable coatings are within the purview of those skilled in the art, as are methods for application of coatings to articles such as medical devices, packaging materials, and textiles. In one embodiment, the coating may include a film-forming polymer. Such coatings may be particularly useful when applied to medical devices. Film-forming polymers which may be utilized in the coating are within the purview of those skilled in the art and include glycolide, lactide, caprolactone, trimethylene carbonate, dioxanones, dioxepanones, etc., and copolymers and combinations thereof. In embodiments, the novel triclosan salts of the present disclosure may be separate from the additional coating. In other embodiments the novel triclosan salts of the present disclosure may be incorporated in the additional coating.

In one embodiment, the film-forming polymer utilized as the additional coating includes a caprolactone containing copolymer as described in U.S. Patent No. 5,716,376, the entire disclosure of which is incorporated by reference herein. Such a caprolactone containing copolymer can be obtained by polymerizing a major amount of epsilon-caprolactone and a minor amount of at least one other copolymerizable monomer or mixture of such monomers in the presence of a polyhydric alcohol initiator.

Monomers which can be copolymerized with epsilon-caprolactone include alkylene carbonates such as trimethylene carbonate, tetramethylene carbonate and/or dimethyl trimethylene carbonate; dioxanones; dioxepanones; absorbable cyclic amides; absorbable cyclic ether-esters derived from crown ethers; hydroxyacids capable of esterification, including alpha hydroxy acids (such as glycolic acid and lactic acid) and beta hydroxyacids (such as beta hydroxybutyric acid and gamma hydroxyvaleric acid); polyalkyl ethers (such as polyethylene glycol); and combinations thereof. In embodiments, glycolide can be utilized as the comonomer in the film-forming polymer.

Suitable polyhydric alcohol initiators which may be utilized in preparing the film-forming polymer include glycerol, trimethylolpropane, 1,2,4-butanetriol, 1,2,6-hexanetriol, triethanolamine, triisopropanolamine, erythritol, threitol, pentaerythritol, ribitol, arabinitol, xylitol, N,N,N',N'-tetrakis(2-hydroxyethyl) ethylenediamine, N,N,N',N'-tetrakis(2-hydroxypropyl)ethylenediamine, dipentaerythritol, allitol, dulcitol, glucitol, altritol, iditol, sorbitol, mannitol, inositol, and the like; with mannitol being useful in some embodiments.

The polyhydric alcohol initiator can be generally employed in small amounts, that is, from about 0.01 to about 5 weight percent of the total monomer mixture, in embodiments from about 0.1 to about 3 weight percent of the total monomer mixture.

Where utilized, the film-forming copolymer can contain from about 70 to about 98 weight percent epsilon-caprolactone derived units, in embodiments from about 80 to about 95 weight percent epsilon-caprolactone derived units, the balance of the copolymer being derived from the other copolymerizable monomer(s), such as glycolide.

In one embodiment, a coating for a medical device can include a film-forming polymer combined with a fatty acid component that contains a fatty acid, a fatty acid salt, or a salt of a fatty acid ester. Suitable fatty acids may be saturated or unsaturated, and include higher fatty acids having more than about 12 carbon atoms. Suitable saturated fatty acids include, for example, stearic acid, palmitic acid, myristic acid and lauric acid. Suitable unsaturated fatty acids include oleic acid, linoleic acid, and linolenic acid. In addition, an ester of fatty acids, such as sorbitan tristearate or hydrogenated castor oil, may be used.

Suitable fatty acid salts include the polyvalent metal ion salts of C₆ and higher fatty acids, particularly those having from about 12 to about 22 carbon atoms, and mixtures thereof. Fatty acid salts including the calcium, magnesium, barium, aluminum, and zinc salts of stearic, palmitic and oleic acids may be useful in some embodiments of the present disclosure. Particularly useful salts include commercial "food grade" calcium stearate which includes a mixture of about one-third C₁₆ and two-thirds C₁₈ fatty acids, with small amounts of the C₁₄ and C₂₂ fatty acids.

Suitable salts of fatty acid esters which may be included in the bioactive coatings of the present disclosure include calcium, magnesium, aluminum, barium, or zinc stearoyl lactylate; calcium, magnesium, aluminum, barium, or zinc palmityl lactylate; and/or calcium, magnesium, aluminum, barium, or zinc olelyl lactylate. In embodiments; calcium stearoyl-2-lactylate (such as the calcium stearoyl-2-lactylate commercially available under the tradename VERV from American Ingredients Co., Kansas City, Mo.) may be utilized. Other fatty acid ester salts which may be utilized include those selected from the group consisting of lithium stearoyl lactylate, potassium stearoyl lactylate, rubidium stearoyl lactylate, cesium stearoyl lactylate, francium stearoyl lactylate, sodium palmityl lactylate, lithium palmityl lactylate, potassium palmityl lactylate, rubidium palmityl lactylate, cesium palmityl lactylate, francium palmityl lactylate, sodium olelyl lactylate, lithium olelyl lactylate, potassium olelyl lactylate, rubidium olelyl lactylate, cesium olelyl lactylate, and francium olelyl lactylate.

Where utilized, the film-forming polymer, such as the caprolactone/glycolide copolymer described above, can be present in an amount from about 45 to about 60 weight percent of the coating and the fatty acid component, such as a fatty acid salt or a salt of a fatty acid ester, can be present in an amount from about 40 to about 55 weight percent of the coating. In embodiments, the film-forming polymer, such as the caprolactone/glycolide copolymer described above, can be present in an amount from about 50 to about 55 weight percent of the coating and the fatty acid component can be present in an amount from about 45 to about 50 weight percent of the coating.

Where a separate coating is present on the article, it should be understood that an antimicrobial solution having the novel triclosan salts in accordance with the present disclosure can be applied before, concurrently with, or after application of the coating. Thus, in some embodiments the antimicrobial solution may be applied to a previously coated article, such as a medical device, packaging material or textile. In other embodiments, the antimicrobial solution may be mixed with the coating composition prior to application to the article. In these embodiments, the coating and antimicrobial solution are applied in a single step.

In other embodiments the antimicrobial solution maybe injected into or onto finished goops through specialized packaging, such as by way of injection ports, and the like. Such ???? include those disclosed in U.S. Patent Application Serial No.: 11/340,912, filed on January 26, 2006, the entire disclosure of which is incorporated by referenced herein.

Where applied as a coating, the silver-triclosan salts of the present disclosure remain attached to the surface of the article during the processing, handling, and storage of the article. This minimizes the loss or transfer of the silver-triclosan salt from an article to any packaging, from any packaging to any article, the environment, etc.

In one embodiment, a medical device treated in accordance with the present disclosure is a suture. Sutures in accordance with the present disclosure may be monofilament or multifilament and may be made of any conventional material, including both bioabsorbable and non-bioabsorbable materials, such as surgical gut, silk, cotton, polyolefins such as polypropylene, polyamides, polyglycolic acids, polyesters such as polyethylene terephthalate and glycolide-lactide copolymers, etc.

In one embodiment, the suture may be made of a polyolefin. Suitable polyolefins include polyethylene, polypropylene, copolymers of polyethylene and polypropylene, and blends of polyethylene and polypropylene. In some embodiments, polypropylene can be utilized to form the suture. The polypropylene can be isotactic polypropylene or a mixture of isotactic and syndiotactic or atactic polypropylene.

In other embodiments, the suture may be made from synthetic absorbable polymers such as those made from glycolide, lactide, caprolactone, alkylene carbonates (i.e., trimethylene carbonate, tetramethylene carbonate, etc.), dioxanones, and copolymers and combinations thereof. One combination which may be utilized includes glycolide and lactide based polyesters, including copolymers of glycolide and lactide.

As noted above, the suture can be monofilament or multifilament. Where the suture is a monofilament, methods for producing such sutures are within the purview of those skilled in the art. Such methods include forming a suture material, such as a polyolefin resin, and extruding, drawing and annealing the resin to form the monofilament.

Where the sutures are made of multiple filaments, the suture can be made using any technique within the purview of one skilled in the art such as, for example, braiding, weaving or knitting. The filaments may also be combined to produce a non-woven suture. The filaments themselves may be drawn, oriented, crinkled, twisted, commingled or air entangled to form yarns as part of the suture forming process.

In embodiments a multifilament suture of the present disclosure can be produced by braiding. The braiding can be done by any method within the purview of those skilled in the art. For example, braid constructions for sutures and other medical devices are described in U.S. Pat. Nos. 5,019,093, 5,059,213, 5,133,738, 5,181,923, 5,226,912, 5,261,886, 5,306,289, 5,318,575,5,370,031, 5,383,387, 5,662,682, 5,667,528, and 6,203,564, the entire disclosures of each of which are incorporated by reference herein. Once the suture is constructed, it can be sterilized by any means within the purview of those skilled in the art.

In some cases a tubular braid, or sheath, can be constructed about a core structure which is fed through the center of a braider. Known tubular braided sutures, including those possessing cores, are disclosed, e.g., in U.S. Pat. Nos. 3,187,752,3,565,077, 4,014,973, 4,043,344, and 4,047,533.

Textiles, including individual fibers and fabrics made of multiple fibers, may be formed and/or coated in a similar manner.

Medical devices and packaging materials in accordance with this disclosure can be sterilized in accordance with techniques within the purview of those skilled in the art.

As noted above, in other embodiments the silver-triclosan salts of the present disclosure may be utilized as an active drug delivery pharmaceutical composition to release the silver ions and/or triclosan in vivo by ion exchange or chelation with endogenous salts. In these embodiments, the silver-triclosan salts may be administered by themselves or in a pharmaceutically acceptable carrier as a pharmaceutical composition. Techniques for formulation and administration of the salts of the present disclosure may be found in "Remington's Pharmaceutical Sciences," Mack Publishing Co., Easton, PA, latest edition. These pharmaceutical compositions can be administered to treat or inhibit infection, either by directly applying the silver-triclosan salts to an infected area or an area where the chance of infection is increased, for example where a drain exits the body or a sutured area. The salts may, in some embodiments, be applied as a liquid or dried powder (lyophilized).

The composition may also be administered topically or parenterally as desired. When administered parenterally, the therapeutic composition should be sterile, pyrogen-free and in a parenterally acceptable solution having due regard for pH, isotonicity, and stability. These conditions are within the purview of those skilled in the art.

Pharmaceutical compositions of the present disclosure may further include suitable known excipients including fillers, disintegrants or effervescent systems, lubricants, glidants, flavors and coloring agents, and other pharmaceutically active agents.

Briefly, dosage formulations of the silver-triclosan salts of the present disclosure may be prepared for storage or administration by mixing the silver-triclosan salts with physiologically acceptable carriers, excipients, or stabilizers. Such materials are nontoxic to the recipients at the dosages and concentrations employed, and may include buffers such as TRIS HCI, phosphate, citrate, acetate and other organic acid salts; antioxidants such as ascorbic acid; low molecular weight (less than about ten residues) peptides such as polyarginine; proteins such as serum albumin, gelatin, or immunoglobulins; hydrophilic polymers such as polyvinylpyrrolidinone; amino acids such as glycine, glutamic acid, aspartic acid, or arginine; monosaccharides, disaccharides, and other carbohydrates including cellulose or its derivatives, glucose, mannose, or dextrins; chelating agents such as EDTA; sugar alcohols such as mannitol or sorbitol; counterions such as sodium and/or nonionic surfactants such as TWEEN, PLURONICS or polyethylene glycol.

When used for in vivo administration, the silver-triclosan salt should be sterile and can be formulated with a carrier according to conventional pharmaceutical practice. Vehicles such as naturally occurring vegetable oils like sesame, peanut, or cottonseed oil or a synthetic fatty vehicle like ethyl oleate or the like may be utilized for administration. Buffers, preservatives, antioxidants and the like can be incorporated according to accepted pharmaceutical practice.

An effective amount of a silver-triclosan salt will depend, for example, upon the microbial infection being treated or prevented, the route of administration, and the condition of the patient.

While the above description contains many specifics, these specifics should not be construed as limitations on the scope of the disclosure herein but merely as exemplifications of particularly useful embodiments thereof. Those skilled in the art will envision many other possibilities within the scope and spirit of the disclosure as defined by the claims appended hereto.

The invention can be described by reference to the following numbered paragraphs:-
1. A salt comprising triclosan and silver, wherein the triclosan is present in an amount from about 40 to about 99% by weight of the salt and the silver is present in an amount from about 1 to about 60% by weight of the salt.
2. A pharmaceutical composition comprising the silver-triclosan salt of paragraph 1.
3. The pharmaceutical composition of paragraph 2, further comprising a pharmaceutically acceptable carrier.
4. An article comprising a silver-triclosan salt, wherein the article is selected from the group consisting of medical devices, packaging materials and textiles.
5. The article of paragraph 4 wherein the medical device is selected from the group consisting staples, clips, drug delivery devices, stents, pins, screws, sutures, prosthetic ligaments, prosthetic tendons, woven mesh, gauze, dressings, and growth matrices.
6. The article of paragraph 4 wherein the packaging material is for a product selected from the group consisting of medical devices, pharmaceuticals, textiles, consumer goods and foods.
7. The article of paragraph 4 wherein the textile is selected from the group consisting of natural fibers, synthetic fibers, blends of natural fibers, blends of synthetic fibers, and blends of natural fibers with synthetic fibers.
8. The article of paragraph 4 wherein the textile is selected from the group consisting of polyesters, polyamides, polyolefins, halogenated polymers, polyester/polyethers, polyurethanes, homopolymers thereof, copolymers thereof, and combinations thereof.
9. The article of paragraph 4 wherein the textile is selected from the group consisting of polyethylene, polypropylene, polybutylene, polyvinyl chloride, polyethylene terephthalate, nylon 6, and nylon 6,6.
10. The article of paragraph 4 wherein the article comprises a polymer having the silver-triclosan salt incorporated therein.
11. The article of paragraph 10 wherein the silver-triclosan salt is present in an antimicrobially-effective amount.
12. The article of paragraph 11 wherein the silver-triclosan salt is present in the article in an amount from about 0.001 to about 25 percent by weight of the article.
13. The article of paragraph 4 wherein the silver-triclosan salt comprises a coating on the article.
14. The article of paragraph 4 wherein the article further comprises a coating comprising a film-forming polymer.
15. The article of paragraph 13 wherein the article comprises a medical device and the silver-triclosan salt comprises from about 0.001 to about 25 percent by weight of the medical device.
16. The article of paragraph 13 wherein the article comprises a packaging material and the silver-triclosan salt comprises from about 0.001 to about 25 percent by weight of the packaging material.
17. The article of paragraph 13 wherein the article comprises a textile and the silver-triclosan salt comprises from about 0.001 to about 25 percent by weight of the textile.
18. A method for making a silver-triclosan salt comprising: combining triclosan with at least one base to form a first triclosan salt; and combining a silver compound with the first triclosan salt to form the silver- triclosan salt.
19. The method of paragraph 18 wherein the base is selected from the group consisting of sodium hydroxide, potassium hydroxide, calcium hydroxide, magnesium hydroxide, aqueous sodium bicarbonate, aqueous sodium carbonate, aqueous calcium carbonate, aqueous potassium carbonate and aqueous potassium bicarbonate.
20. The method of paragraph 18 wherein the silver compound is a silver salt selected from the group consisting of silver acetate, silver ascorbate, silver benzoate, silver bromide, silver carbonate, silver chloride, silver citrate, silver gluconate, silver iodate, silver iodide, silver lactate, silver laurate, silver nitrate, silver oxide, silver palmitate, silver phosphate, silver propionate, silver salicylate, silver sulfate, silver laurel sulfate, and combinations thereof.
21. The method of paragraph 18 wherein the silver compound is selected from the group consisting of colloidal silver, silver protein, silver sulfadiazine, silver arsphenamine, zinc-silver allantoinate, and combinations thereof.
22. The method of paragraph 18 wherein the triclosan, base and silver compound are combined in a solution and heated to a temperature from about 60°C to about 180°C, for a period of time from about 2 minutes to about 24 hours.
23. The method of paragraph 22 wherein the silver-triclosan salt is recovered from the solution by a method selected from the group consisting of distillation, filtration, and centrifugation.
24. The method of paragraph 18 wherein triclosan is present in the silver-triclosan salt in an amount from about 40 to about 99 percent by weight of the total weight of the silver-triclosan salt, and the silver ion is present in an amount from about 1 to about 60 percent by weight of the total weight of the silver-triclosan salt.
25. The method of paragraph 18 further comprising complexing the silver- triclosan salt with chlorhexidine or its salts to form a silver-chlorhexidine-triclosan complex.
26. The method of paragraph 25 wherein the chlorhexidine salts are selected from the group consisting of chlorhexidine acetate, chlorhexidine gluconate, chlorhexidine hydrochloride, chlorhexidine sulfate, and combinations thereof.

## Claims

1. An article comprising a silver-triclosan salt, wherein the article is selected from the group consisting of medical devices and packaging materials, and wherein the triclosan is present in an amount from about 40% to about 99% of the weight of the salt and the silver is present in an amount from about 1 to about 60% by weight of the salt.

2. The article of claim 1 wherein the medical device is selected from the group consisting staples, clips, drug delivery devices, stents, pins, screws, prosthetic ligaments, prosthetic tendons, woven mesh, gauze, dressings, and growth matrices.

3. The article of claim 1 wherein the packaging material is for a product selected from the group consisting of medical devices, pharmaceuticals, textiles, consumer goods and foods.

4. The article of any preceding claim wherein the article comprises a polymer having the silver-triclosan salt incorporated therein.

5. The article of any preceding claim wherein the silver-triclosan salt is present in an antimicrobially-effective amount.

6. The article of any preceding claim wherein the silver-triclosan salt is present in the article in an amount from about 0.001 to about 25 percent by weight of the article.

7. The article of any preceding claim wherein the silver-triclosan salt comprises a coating on the article, or wherein the article further comprises a coating comprising a film-forming polymer.
